**Europäisches Patentamt**

**European Patent Office**

**Office européen des brevets**

(19)

(11) Veröffentlichungsnummer: **0 019 044**
**B1**

(12) ## EUROPÄISCHE PATENTSCHRIFT

(45) Veröffentlichungstag der Patentschrift:
**23.06.82**

(51) Int. Cl.³: **A 61 F 1/03**

(21) Anmeldenummer: **80101211.3**

(22) Anmeldetag: **10.03.80**

(54) **Schaftteil einer Hüftgelenkendoprothese und Verfahren zur Herstellung desselben.**

(30) Priorität: **19.05.79 DE 2920336**

(43) Veröffentlichungstag der Anmeldung:
**26.11.80 Patentblatt 80/24**

(45) Bekanntmachung des Hinweises auf die Patenterteilung:
**23.06.82 Patentblatt 82/25**

(84) Benannte Vertragsstaaten:
**AT CH FR GB**

(56) Entgegenhaltungen:
**DE-A-2 049 111**
**DE-A-2 621 123**
**DE-A-2 805 868**
**FR-A-2 360 295**
**FR-A-2 361 093**
**ENGINEERING IN MEDICINE, Band 7, Nr. 3, Juli 1978, G. BARBERI et al.: »Flexural coupling of hip-prosthesis stem and femur: a simple method of theoretical analysis«, Seiten 172—178**

(73) Patentinhaber: **SIGRI ELEKTROGRAPHIT GMBH,**
**Werner von Siemens-Strasse 18, D-8901 Meitingen (DE)**

(72) Erfinder: **Rose, Philip, Dr. rer. nat.,**
**Abt-Königsdorferstrasse 18, D-8850 Donauwörth (DE)**
Erfinder: **Gerstenberger, Friedrich, Prof. Dipl.-Ing.,**
**Grabenstrasse 11, D-7321 Dürnau (DE)**
Erfinder: **Loos, Wolfgang, Ing. grad., Studentenweg 6,**
**D-7932 Munderkingen (DE)**

Schaftteil einer Hüftgelenkendoprothese und Verfahren zur Herstellung desselben

Die Erfindung betrifft den Schaftteil einer Hüftgelenkendoprothese, der aus mit Fasern verstärktem Werkstoff besteht.

Faserverstärkte Werkstoffe werden seit längerer Zeit zur Herstellung von menschliche und tierische Organe ersetzenden Implantaten verwendet (z. B. DE-A-1 902 700). Die Verwendung von kohlenstoffaserverstärktem Kohlenstoff als Werkstoff für Prothesen und besonders für Prothesenschäfte ist beispielsweise durch die DE-A1-2 621 123 bekannt. Vorteile dieses Werkstoffs sind seine biologische Kompatibilität, d. h., lebende tierische und menschliche Gewebe werden nicht im Kontakt mit Kohlenstoff verändert, und die physikalische Kompatibilität, unter der eine Angleichung der elastischen Eigenschaften des Werkstoffs an die Elastizität des Knochens zu verstehen ist. Die Elastizität von Prothesen aus kohlenstoffaserverstärktem Kohlenstoff kann in einfacher Weise durch Änderungen des Gehalts an Kohlenstoffasern oder auch durch Änderungen der Faserorientierung eingestellt werden und der Elastizität des Knochens angepaßt werden. Durch die DE-A1-2 621 123 ist es darüber hinaus bekannt, die den Werkstoff verstärkenden Kohlenstoffasern in mehreren gegeneinander geneigten Scharen anzuordnen, wobei die Längserstreckung der Fasern innerhalb einer Schar mit einer Hauptbelastungsrichtung zusammenfällt, um die Ein- und Ableitung von Kräften zu verbessern.

Die Bedingungen der Isoelastizität und der gezielten Ein- und Ableitung der Kräfte reichen jedoch nicht aus, Relativbewegungen zwischen dem Prothesenschaft und dem anliegenden Knochengewebe vollständig zu verhindern und auch nicht derartig zu begrenzen, daß eine Lockerung des Prothesenschafts ausgeschlossen ist. Dieses nicht völlig befriedigende Verhalten der bekannten Prothesenschäfte aus kohlenstoffaserverstärktem Kohlenstoff wird zu einem Teil durch die Variationsbreite des lebenden Knochengewebes bedingt, vor allem aber dadurch, daß das Trägheitsmoment des Prothesenschafts über die Schaftlänge nicht konstant ist, sondern sich für jede Schaftform in gesetzmäßiger Weise ändert. Der Erfindung liegt daher die Aufgabe zugrunde, die Wirkung örtlich verschiedener Trägheitsmomente zu kompensieren und im gesamten Belastungsbereich der Prothese für jede Last eine der Verformung des anliegenden Knochengewebes identische Verformung des Prothesenschafts zu erreichen.

Die Aufgabe wird mit einem Prothesenschaftteil der eingangs genannten Art dadurch gelöst, daß der Elastizitätsmodul des faserverstärkten Werkstoffs von der Spitze des Schafts zu dem entgegengesetzten Ende abnimmt.

Die Erfindung beruht auf der Erkenntnis, daß Relativbewegung zwischen Prothesenschaft und Knochengewebe durch einen über die Länge des Prothesenschafts einen konstanten Elastizitätsmodul aufweisenden Werkstoff oder durch faserverstärkte, die Fasern in Scharen oder Bündeln enthaltende Werkstoffe nicht vermieden werden können, sondern allein durch einen Werkstoff, dessen Elastizitätsmodul von der Schaftspitze zum entgegengesetzten Ende abnimmt, d. h., durch eine Prothese, deren Elastizitätsmodul einen Gradienten aufweist. Dabei ist es offensichtlich nicht erforderlich, daß das Trägheitsmoment jedes einzelnen Schaftquerschnitts durch den Elastizitätsmodul des Schaftwerkstoffs an jeder Stelle genau ausgeglichen wird, es reicht vielmehr ein dem Verlauf der Trägheitsmomente angepaßter Gradient der Elastizität aus. Für das Trägheitsmoment der schaftförmigen Prothese gilt angenähert:

$$I = 1,5 \cdot 10^5 - 2,2 \cdot 10^4 \ln k,$$

wobei k der axiale Abstand ist.

Die Erfüllung der Bedingung

$$\varepsilon_P = \varepsilon_K$$

($\varepsilon$ = Verformung, P = Prothese, K = Knochen) erfordert entsprechend eine gleichsinnige Änderung des Elastizitätsmoduls des Prothesenschafts, dessen Betrag aus dem mittleren Elastizitätsmodul des Knochens und den Änderungen des Knochenquerschnitts über die Schaftlänge mit ausreichender Genauigkeit berechnet werden kann. Beispielsweise sollte bei einem Knochen mit einem mittleren Modul von 16,5 kN/mm² für drei Schnittebenen eines dem Knochen anliegenden Prothesenschafts, deren größter Umfangsabstand von der Schaftachse 10, 16 bzw. 20 mm beträgt, der Elastizitätsmodul des Prothesenwerkstoffs in dieser Ebene etwa 81, 46 und 30 kN/mm² betragen.

Wenngleich gegenüber den bekannten Prothesen ein wesentlicher Fortschritt bereits durch einen nichtquantifizierten Gradienten erreicht wird, empfiehlt es sich, unter Nutzung der bekannten Proportionalität von Elastizitätsmodul und dem Volumenanteil der Verstärkungsfasern den Gradienten dem Verlauf des Trägheitsmoments anzupassen.

In dem Prothesenschaft wird der Modul-Gradient bevorzugt dadurch eingestellt, daß der Anteil der Verstärkungsfasern in der Matrix von der Schaftspitze zum Schaftende abnimmt.

Nach einer weiteren bevorzugten Ausbildung der Prothese besteht der Prothesenschaft aus mehreren Schichten, die Verstärkungsfasern in paralleler Orientierung enthalten, wobei die Fasern wenigstens einiger benachbarter Schichten eine verschiedene Orientierung aufweisen. Bei dieser Ausbildung ist der Anteil von Schichten, die Kohlenstoffasern einer bestimmten Orientierung enthalten, der Dicke des Prothesenschafts proportional, so daß sich der Elastizitätsmodul analog mit der Schaftdicke ändert.

Als Werkstoff kann Kohlenstoff und als Verstärkungsfasern können Kohlenstofffasern verwendet werden.

Zum Herstellen eines Prothesenschafts werden mit einem härtbaren Kunstharz imprägnierte Fasern, insbesondere Kohlenstofffasern, in zueinander paralleler Orientierung in Schichten ausgelegt, mehrere gegeneinander verdrehte Schichten übereinandergestapelt und gegebenenfalls unter einem erhöhten Druck zum Härten und Carbonisieren auf eine Temperatur zwischen etwa 120 und 200°C und dann unter inerten Bedingungen auf etwa 1000°C erhitzt. Unter dem Begriff »Kohlenstofffasern« sind alle im wesentlichen aus dem Element Kohlenstoff bestehenden, durch Pyrolyse natürlicher und synthetischer faserförmiger Stoffe hergestellten Fasern zu verstehen, z. B. auch Graphitfasern. Die Fasern werden in Form von Garnen oder Rovings oder als Flächengebilde, wie z. B. Geweben, oder auch Kurzschnittfasern verarbeitet.

Nach einem anderen Verfahren werden insbesondere Kohlenstofffasern in unidirektionaler Anordnung aufeinandergestapelt und in gesetzmäßiger Weise gegeneinander verdreht. Beispielsweise beträgt die Faserorientierung in einer mittleren Schicht 0°, in den anliegenden Schichten ±40°, in den darauffolgenden Schichten 90° und so fort. Der Schichtstapel wird wie oben beschrieben thermisch behandelt und nach der Überführung in Kohlenstoff ein Prothesenschaft in Richtung 0° herausgearbeitet. Im Bereich der Schaftspitze bleibt dabei nur die mittlere Schicht erhalten und mit zunehmender Dicke des Schafts sind Schichten mit ±40° Fasern und schließlich mit 90° Fasern enthalten. Der Elastizitätsmodul ergibt sich dabei aus dem Anteil der verschieden orientierten Fasern.

## Patentansprüche

1. Schaftteil für eine Hüftgelenkendoprothese aus einem mit Fasern verstärkten Werkstoff, dadurch gekennzeichnet, daß der Elastizitätsmodul des faserverstärkten Werkstoffs von der Spitze des Schafts zu dem entgegengesetzten Schaftende abnimmt.

2. Schaftteil nach Patentanspruch 1, dadurch gekennzeichnet, daß in der Prothese der Volumenanteil der Verstärkungsfasern von der Spitze zum Schaftende abnimmt.

3. Schaftteil nach den Patentansprüchen 1 und 2, dadurch gekennzeichnet, daß der Schaftteil aus mehreren, Verstärkungsfasern in paralleler Orientierung entaltenden Schichten besteht und die Fasern wenigstens einiger benachbarter Schichten verschieden zueinander orientiert sind.

4. Schaftteil nach den Patentansprüchen 1 bis 3, dadurch gekennzeichnet, daß der Werkstoff Kohlenstoff und die Verstärkungsfasern Kohlenstofffasern sind.

5. Verfahren zum Herstellen eines Prothesenteils nach den Patentansprüchen 1 bis 4, dadurch gekennzeichnet, daß mit einem Kunstharz imprägnierte Kohlenstofffasern zu Schichten ausgelegt werden, in denen die Fasern parallel zueinander orientiert sind, mehrere gegeneinander verdrehte Schichten übereinandergestapelt werden und der Stapel unter einem erhöhten Druck zum Härten und Carbonisieren des Kunstharzes einer thermischen Behandlung unterzogen und aus dem Rohling das Prothesenteil gearbeitet wird.

## Claims

1. Stem part of a hip joint endoprosthesis consisting of a fibre-reinforced material characterized by a Young's Modulus of the stem which decreases from the tip of the stem to its opposite end adjacent to the joint.

2. Stem part according to claim 1 which comprises a decrease of fibre content by volume from the tip of the stem to its opposite end.

3. Stem part according to claim 1 and 2 which comprises a plurality of sheets containing aligned reinforcing fibres, the alignments of at least some neighbouring sheets being different from eachother.

4. Stem part according to claim 1 to 3 which comprises carbon fibre reinforced carbon.

5. Method for producing fibrereinforced stem parts of hip joint endoprosthesis according to claim 1 to 4 which comprises coating the carbon fibres with a resin, aligning and laying out the fibres into layers, stacking the layers and heating the stack at an elevated pressure to cure and to carbonize the resin and machining the carbon body.

## Revendications

1. Tige fémorale d'une endoprothèse de la hanche, en matériau renforcé par des fibres, tige caractérisée en ce que le module d'élasticité du matériau renforcé par des fibres, décroît de la pointe de la tige jusqu'à l'extrémité opposée de cette tige.

2. Tige selon la revendication 1, caractérisée en ce que dans la prothèse, la proportion volumique des fibres de renforeement décroît de la pointe jusqu'à l'extrémité de cette tige.

3. Tige selon l'une des revendications 1 ou 2, caractérisée en ce qu'elle est constituée par plusieurs couches contenant les fibres de renforcement à orientation parallèle, et que les fibres d'au moins quelques couches adjacentes sont orientées différemment les unes par rapport aux autres.

4. Tige selon l'une quelconque des revendications 1 à 3, caractérisée en ce que le matériau est du carbone et que les fibres de renforcement sont des fibres de carbone.

5. Procédé pour la fabrication d'une pièce de prothèse selon l'une quelconque des revendications 1 à 4, caractérisée en ce que des fibres de carbone imprégnées de résine synthétique sont disposées en couches dans lesquelles les fibres sont orientées parallèlement, entre elles, que plusieurs couches d'orientation différentes somt empilées les unes sur les autres, et que la pile est soumise à un traitement thermique, sous pression élevée, pour réaliser le durcissement et la carbonisation de la résine, et qu'à partir de l'ébauche, la pièce de prothèse est obtenue par usinage.